# EUROPEAN PATENT APPLICATION

(11) **EP 1 964 509 A1**
(43) Date of publication of application: **03.09.2008**
(21) Application number: 08003464.8
(22) Date of filing: 26.02.2008
(51) Int. Cl.: A61B 1/005, A61B 1/012

(54) **Therapeutic system used with steps for approaching lesion using overtube**

(30) Priority: 01.03.2007 US 712696
(71) Applicant: Olympus Medical Systems Corp., Tokyo 151-0072 (JP)
(72) Inventor: Motai, Kousuke, Tokyo 151-0072 (JP); Onuki, Yoshio, Tokyo 151-0072 (JP); Kura, Yasuhito, Tokyo 151-0072 (JP)
(74) Representative: von Hellfeld, Axel

(57) **Abstract**

A therapeutic system is provided, which is preferable to a treatment of the pancreatic duct and binary duct for Roux-en-Y gastrectomy patients. The therapeutic system is provided with a catheter equipped with an Imaging device outputting a signal in reply to imaging, an overtube to be inserted into inside a patient, and a display displaying images based on the signal outputted from the imaging device. The overtube has an insertion channel into which the catheter is inserted, a therapeutic instrument channel into which a therapeutic instrument is inserted, an elevating base formed in an opening located at a distal end of, at least, the therapeutic-instrument channel, and an operation device to operate the elevating base. The elevating base is formed to allow a distal end portion of the therapeutic instrument to be mounted on the elevating base to elevate the distal end portion.

## Description

### Background of the Invention

### (Technical field of the Invention)

The present invention relates to a therapeutic system which are preferable to a treatment of pancreatic duct and biliary duct of a patient who has the Roux-en-Y reconstructive operation.

### (Related Art)

Endoscopic examinations and treatments for biliary disease and pancreas disease, such as biliary tract cancer, pancreas cancer, cholelithiasis, and common bile duct stones, are progressing at a rapid pace. Compared to the conventional surgical treatments, these examinations and treatments are less invasive and less of a burden on patients. The techniques for these examinations and treatments include endoscopic retrograde cholangiopancreatography (ERCP) or Endoscopic sphincterotomy. These endoscopic examinations and treatments are on the progress to applications to postgastrectomy cases.

Conventionally, in cases where a patient (an object being treated) who had the Roux-en-Y reconstructive operation needs a treatment of pancreatic duct and biliary duct on the ERCP, a side-viewing endoscope and/or an oblique-viewing endoscope are used.

For example, in the literature 1 "R. Hirai et al., "An Oblique-viewing Endoscope with an Overtube Facilitate Bile Duct Stone Removal in Roux-en-Y Gastrectomy Patients," Japanese Gastroenterological Endoscopy, 2006; 48: 212-217," there is a report that ERCP with an overtube for endoscopy of the small intestine was successfully performed in the treatment of common bile duct stones for patients with Roux-en-Y gastrectomy. The ERCP was conducted with the use of an oblique-viewing endoscope. In this ERCP, the following operations were repeated such that the endoscope was stretched for shorten the gastrointestinal tract, before the overtube is pushed to advance. This is able to suppress the shortened gastrointestinal tract from sagging and, in this suppressed state, the endoscope is made to advance. Although the number of cases is few, it is reported that the duodenum papilla could be reached in each case.

However, it is difficult to use the technique of using the side-viewing endoscope and/or the oblique-viewing endoscope in that its insertion tube and/or an overtube are made to pass along the jejunum-anastomotic bent portion. As a result, it takes more time to approach the Vater's papilla and it takes longer to perform a treatment of pancreatic duct and biliary duct. In addition, it is required for doctors to have considerable skills to pass the insertion tube of an endoscope and/or an overtube through the jejunum-anastomotic bent portion.

### Summery of the Invention

The present invention has been made in consideration of the problems that the foregoing conventional technique has confronted, and, in particular, has an object of making it easier to approach the Vater's papilla of a patient who had Roux-en-Y gastrectomy.

According to the present invention, as one aspect thereof, there is provided a therapeutic system preferable to performing a treatment of pancreatic duct and biliary duct of a patient who had Roux-en-Y gastrectomy. This therapeutic system comprises a catheter equipped with an imaging device outputting a signal based on imaging; an overtube to be inserted into inside an object; and a display displaying images based on the signal outputted from the imaging device. The overtube has an insertion channel into which the catheter is inserted, a therapeutic instrument channel into which a therapeutic instrument is inserted, an elevating base formed in an opening located at a distal end of, at least, the therapeutic-instrument channel, and an operation device to operate the elevating base.

Further, as another aspect, the present invention provides a therapeutic method that uses a catheter equipped with an imaging device and an overtube having a bendable distal end portion equipped with an elevating base to elevate the catheter and a further elevating base to elevate a therapeutic instrument. This therapeutic method includes a first step for inserting the catheter into a catheter channel of the overtube; a second step for orally inserting both the catheter and the overtube into a gastric region of a patient who had a Roux-en-Y reconstructive operation, during which time an image obtained by the imaging device is observed, and then for curving the overtube to pass both the catheter and the overtube along a jejunum-to-jejunum anasto region and a jejunum-anastomotic bent portion so as to advance to a duodenum; and a third step for positionally fixing the overtube relatively to the duodenum when both the catheter and the overtube reach a position near a Vater's papilla of the duodenum.

### Brief Description of the Drawings

In the accompanying drawings:
Fig. 1 is a schematic diagram outlining the whole structure of a therapeutic system according to a first embodiment of the present invention;
Fig. 2 is a plan view showing the configuration of the distal end portion of an overtube adopted by the therapeutic system;
Fig. 3A is a sectional view showing the distal end portion of the overtube, taken along an A-A line in Fig. 2;
Fig. 3B is a sectional view showing the distal end portion of the overtube, taken along an A'-A' line in Fig. 2;
Fig. 3C is a sectional view showing the distal end portion of the overtube, taken along an A"-A" line in Fig. 2;
Fig. 4 is a sectional view explaining the operations of an elevating base embedded in the distal end portion of the overtube;
Fig. 5 is a sectional view taken along an F-F line in Fig. 1;
Figs. 6A, 6B and 6C are illustrations pictorially showing elevating patterns of both a catheter and a therapeutic instrument 13, which illustrations are obtained when the overtube is viewed from a distal end side of the distal end portion along an axial direction thereof;
Fig. 7 is a plan view showing a catheter-inserting port formed at a handheld operating portion of the overtube;
Fig. 8 is a sectional view taken along a B-B line in Fig. 7;
Figs. 9, 10 and 11 are sectional views taken along C-C, D-D, and E-E lines in Fig. 7, respectively;
Figs. 12A, 12B and 12C are views explaining operations to lock the overtube of the catheter;
Figs.13A - 13D are views respectively illustrating how an overtube with a catheter inserted therethrough is made to approach the Vater's papilla;
Fig. 14A is a view explaining how the distal end portion of the overtube is positionally fixed in the duodenum and how the Vater's papilla is side-viewed;
Fig. 14B exemplifies a monitor image obtained under the positional relationship shown in Fig. 14A;
Fig. 15A is another view explaining how the distal end portion of the overtube is positionally fixed in the duodenum and how the Vater's papilla is side-viewed;
Fig. 15B exemplifies a monitor image obtained under the positional relationship shown in Fig. 15A;
Fig. 16A is another view explaining how the distal end portion of the overtube is positionally fixed in the duodenum and how the Vater's papilla is side-viewed;
Fig. 16B exemplifies a monitor image imaged under the positional relationship shown in Fig. 16A;
Fig. 17 is a side view, partly cut, which explains a mechanism locking a catheter in a catheter-inserting port in a modification;
Figs. 18A and 18B are partial sectional views each explaining operations of the modified structure shown in Fig. 17;
Fig. 19 is a side view, partly cut, which explains a mechanism locking a catheter in a catheter-inserting port in another modification;
Fig. 20 is a schematic diagram outlining the whole structure of a therapeutic system according to a second embodiment of the present invention;
Fig. 21 is a partial sectional view showing the distal end portion of an overtube employed by the therapeutic system according to the second embodiment;
Figs. 22A and 22B are views each explaining operations of the distal end portion of the overtube shown in Fig. 21;
Figs. 23 and 24 are partial sectional views each explaining operations of the distal end portion of an overtube according to another modification;
Figs. 25 and 26 are partial sectional views each explaining operations of the distal end portion of an overtube according to another modification;
Fig. 27 is a side view explaining an overtube with a marking, which overtube is according to another modification;
Fig. 28 is a partial perspective view showing the appearance of the distal end portion of an overtube according to another modification; and
Fig. 29 is a partial sectional view taken along the axial direction of the distal end portion shown in Fig. 28.

### Detailed Description of Preferred embodiments

Hereinafter, with reference to the accompanying drawings, various embodiments will now be described of a therapeutic method including an approach step to the Vater's papilla of a patient who had the Roux-en-Y gastrectomy (reconstructive operation) and a therapeutic system for performing a treatment of the pancreatic duct and biliary duct by the therapeutic method, which method and system are according to the present invention.

### (First embodiment)

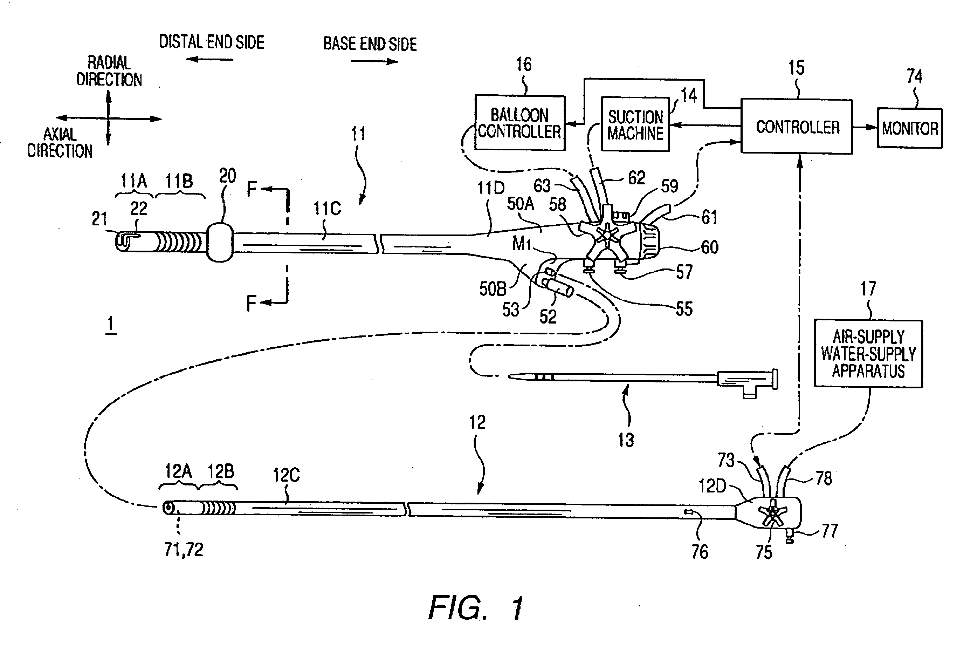

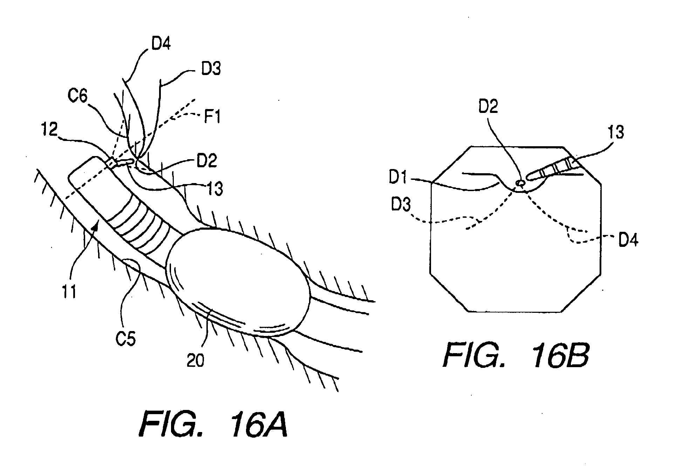

Referring to Figs. 1 - 16A and 16B, a first embodiment will now be described.

A therapeutic system 1 according to the first embodiment is provided with an overtube 11 inserted into a body cavity of a patient (body to be examined), a catheter 12 equipped with imaging means and inserted into the overtube 1 for use, a therapeutic instrument 13 inserted through the overtube 11 for the treatment of pancreatic duct and billary duct, a suction machine 14, a controller 15, a balloon controller 16, and an air-supply water-supply unit 17.

At first, the overtube 11 will now be described. This overtube 11 is produced to facilitate the treatment of the pancreatic duct and biliary duct to be performed with the Vater's papilla of a patient who had undergone the Roux-en-Y reconstructive operation. However, the applications will not be limited to the treatment of the pancreatic duct and biliary duct, but the overtube is applicable to therapies that need to convey a therapeutic instrument to a desired therapeutic position through bent portions of a body cavity in a patient, which bent portions include for example a jejunum-anastomotic bent portion reconstructed by the Roux-en-Y reconstructive operation.

### (Overtube)

As shown In Fig. 1, the overtube 11 is shaped into an elongated form as a whole and similar in shape to an endoscope.

The directional relationships, which are necessary in the following description, will now be defined. The head portions of the overtube 11 and catheter 12, which are put, as a first part thereof, into the object being treated, are called "distal end" or "distal end side" (i.e., the left distal ends in Fig. 1), while the opposite side end to those distal ends, which are handled by an operator, is called "base end" or "base end side" (i.e., the right distal ends In Fig. 1). The longitudinal direction provided by the elongated shape of the overtube 11 is called "axial direction (or longitudinal direction in some cases," and directions along a plane perpendicular to the axial direction are called "radial direction." As will be described later, a large axial part of the overtube 11 is placed within a body cavity of the object when being used, so that the overtube 11 has flexibility that enables free bending actions.

The overtube 11 is a substantially cylindrical device in which a distal end portion 11A, a bendable portion 11B, a flexible tubular portion 11C, and a handheld operating portion 11D are integrated as one tube in this order from the distal end side to the base side. However, to mount various ports and switches to the handheld operating portion 11D, this portion is formed to be slightly thicker than the remaining portions. The cylindrical portions of this overtube 11 are made of resin material (for example, silicone, polyurethane, thermoplastic elastomer, or fluorinated resin). A balloon 20 is provided on the outer surface of the overtube 11 and positioned close to the bendable portion 11B. This balloon 20 is also part of the overtube 11.

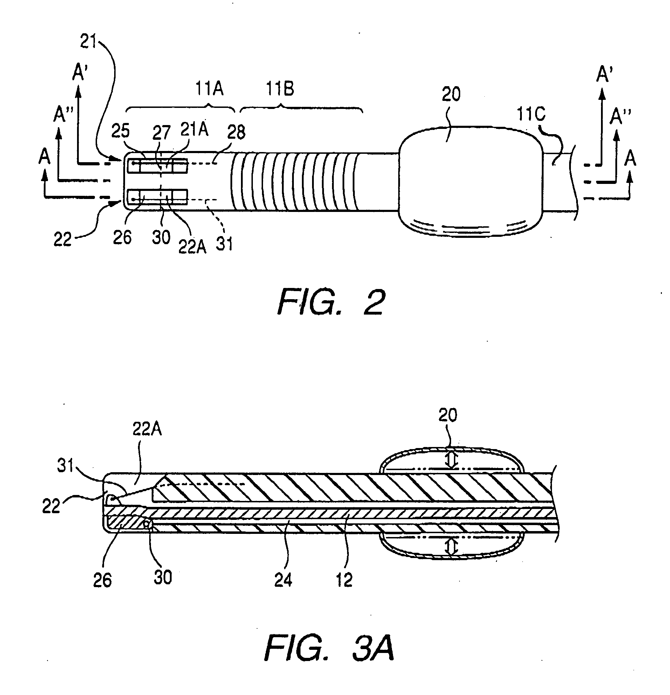

The distal end portion 11A is formed as a rigid portion of a predetermined length, which is from the distal face of the catheter, and is provided with various openings for water supply, air supply and suction, though not shown, as well as a therapeutic-instrument opening 21 and a catheter opening 22 formed respectively to pass through inside the portion along the axial direction thereof. Both openings 21 and 22 for the therapeutic instrument and catheter include slits 21A and 22A, which are part of the openings, as shown in Fig. 2. The slits enable both openings 21 and 22 to open, at their distal ends, not only in the axial direction but also in an angular range of, at least, approximately 90 degrees between the axial direction and a radial direction perpendicular to the axial direction.

Of both openings 21 and 22, one opening 21 communicates with a port formed at the handheld operating portion 11D via a channel formed through the bendable portion 11B, flexible tubular portion 11C, and handheld operating portion 11D. This insertion channel 23 is formed to allow the therapeutic instrument 13 to be Inserted through this channel. Thus the distal end of the therapeutic instrument 13 inserted through the insertion channel 23 reaches the opening 21, and can be located outside this opening 21. Similarly to this, the other opening 22 is made to communicate with another port formed at the handheld operating portion 11D via another channel formed through the bendable portion 11B, flexible tubular portion 11C, and handheld operating portion 11D. This insertion channel 24 is formed to allow the catheter 12 to be inserted through this channel. Thus the distal end of the catheter 12 inserted into the insertion channel 24 reaches the opening 22, and can be located outside the opening 22.

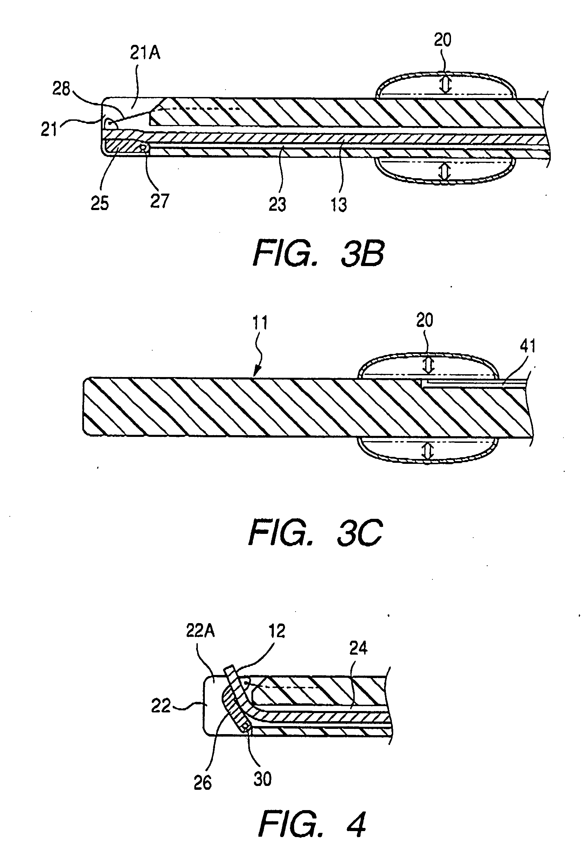

As shown in Figs. 2-4, elevating bases 25 and 26 are disposed in both openings 21 and 22. Practically, in the therapeutic-instrument opening 21, the elevating base 25 for therapeutic instruments is disposed, so that this elevating base 25 is able to elevate the distal end portion of the therapeutic Instrument 13 which has passed through the therapeutic-Instrument channel 23 (in particular, refer to Fig. 3B). This elevating base 25 is a member rotatable on a fixing pin 27 serving as a fulcrum and is formed to rotate on the fixing pin 27, i.e., to be elevated toward a perpendicular standing direction within the opening 21 by pulling a wire 28 attached to a distal side surface of the elevating base 25. The wire 28, which is placed in a wire channel (not shown) formed through the distal end portion 11A, bendable portion 11B, and flexible tubular portion 11C, is linked to the handheld operating portion 11D. Thus, turning an elevating lever (described later) formed at the handheld operating portion 11D enables the wire to be pulled.

In a similar manner to the above, the elevating base 26 for the catheter is disposed in the opening 22 for the catheter, in which the elevating base 26 is formed to elevate the distal end portion of the catheter 12 inserted through the catheter channel 24 (in particular, refer to Figs. 3A and 4). This elevating base 26 is a member rotatable on a fixing pin 30 serving as a fulcrum and is formed to rotate on the fixing pin 30, i.e., to be elevated toward the perpendicular standing direction within the opening 22 by pulling a wire 31 attached to a distal side surface of the elevating base 26. The wire 31, which is placed in a wire channel (not shown) formed through the distal end portion 11A, bendable portion 11B, and flexible tubular portion 11C, is linked to the handheld operating portion 11D. Thus, turning an elevating lever (described later) formed at the handheld operating portion 11D enables the wire to be pulled.

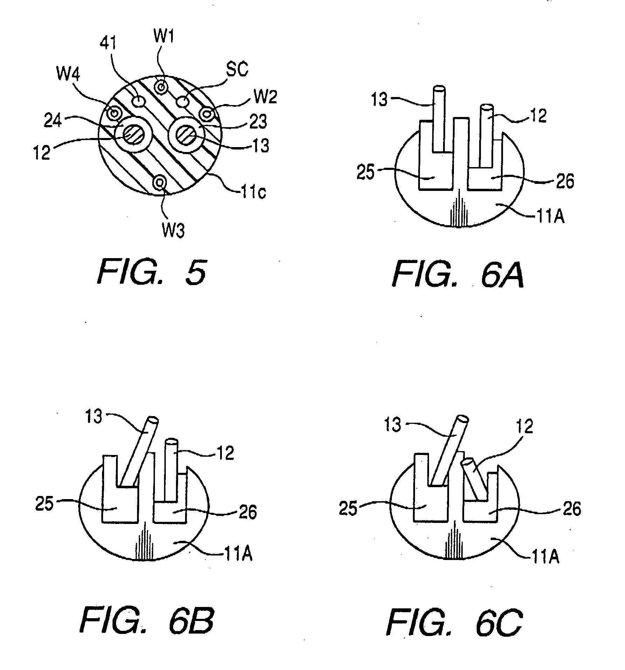

Additionally, in both openings 21 and 22, the actions of the therapeutic instrument 13 and catheter 12 elevated by the respective elevating bases 25 and 26 can be patterned as shown in Figs. 6A to 6C. The elevating pattern 1 shown in Fig. 6A is for elevating the therapeutic instrument 13 and catheter 12 straight and upward along the planes in their axial directions, respectively. In this pattern, the therapeutic instrument 13 and catheter 12 are elevated in a state where the therapeutic instrument and catheter are almost parallel with each other. In contrast, in the elevating pattern 2 shown in Fig. 6B, the catheter 12 is elevated straight and upward along the plane in the axial direction thereof, while the therapeutic instrument 13 is elevated upward, but obliquely so as to be closer to an overtube central axis than the axial direction thereof, that is, so as to approach the catheter 12. Moreover, in the elevating pattern shown in Fig. 6C, the catheter 12 and the therapeutic instrument 13 are elevated upward, but obliquely so as to be closer to the overtube central axis than each of the axial directions thereof, that is, so as to approach the therapeutic instrument 13 and the catheter 12. These elevating patterns 2 and 3 are realized by elevation-angle changing mechanisms (not shown) provided to the elevating bases 25 and 26, for instance.

These elevating patterns 2 and 3 make it possible that the respective distal ends of the catheter 12 and the therapeutic instrument 13 are approached more closely to the center of an object being observed and located thereat, whereby accurate observation and treatment can be done. The bendable portion 11B is formed to integrally couple the distal end portion 11A and the flexible tubular portion 11C to each other. The bendable portion 11B is formed such that, responsively to commands given at the handheld operating portion 11D, the bendable portion 11B is bent freely, for example, in the up, down, right and left four directions directed when viewing is along the overtube axial direction. Pulling and relaxing operations necessary for those active bending actions are provided by, for example, not-shown operating wires connecting a later-described operating lever 58, placed at the handheld operating portion 11D, and the bendable portion 11B. By rotating and operating the operating lever 58, the operating wires are pushed and pulled, resulting in that the bendable portion 11B can be bent in a desired one among the four directions.

The flexible tubular portion 11C is a tubular member integrally connecting the bendable portion 11B and the handheld operating portion 11D to each other and is formed to be flexible depending on force applied in the axial and radial direction thereof. Inside the flexible tubular portion 11C, there are formed various channels (ducts) (refer to Fig. 5) in the axial direction, which channels extend from the various openings of the distal end portion 11A via the bendable portion 11B. Those channels reach the handheld operating portion 11D.

In Fig. 5, a reference SC shows a suction channel and references W1 - W4 show channels though which wires for bending the bendable portion 11B are inserted.

On the outer surface of the flexible tubular portion 11C, the foregoing balloon 20 is loaded. The loading position Is set to be a predetermined position which is near the bendable portion 11B. Incidentally the loading position of this balloon 12 is not necessarily limited to the outer surface of the flexible tubular portion 11C, but may be decided to be, for example, on the outer surface of the distal end portion 11A.

The balloon 20 is a thin, expandable and shrinkable pouch member made of thin flexible resin materials (such as silicone, latex, polyurethane, nylon and thermoplastic resin). Part of this pouch member is secured airtightly on the outer surface of the flexible tubular portion 11C. This pouch member, that is, the balloon 12 has an inner space which communicates with a balloon air-supply channel 41 formed inside the flexible tubular portion 11C (refer to Fig. 3C). Under the operations of a balloon controller 16, the air-supply channel 41 is able to supply and exhaust, for example, air which serves as fluid. The balloon 12 can be expanded by supplying the air and exhausted by exhausting the air. The shrunk state is shown by imaginary lines in Figs. 3A - 3C. When this balloon 12 is shrunk, the outer portion of the flexible tubular portion 11C at which the balloon 12 is located becomes almost flat. When being expanded, the balloon 12 allows part of the flexible tubular portion 11C to form a torus shape thereof expanded by a predetermined length in the radial direction. This expanded state is shown by solid lines in Figs. 3A - 3C. The balloon 12 may be expanded and shrunk by using a syringe handled by an operator.

The handheld operating portion 11D comprises a cylindrical portion 50A and a bulge portion 50B, which cylindrical portion 50A is approximately cylindrical and has a proper radius easier for operators to hold it by hand and which bulge portion 50B is integral with the cylindrical portion 50A and bulges outwardly and obliquely from a predetermined position on the distal end side of the cylindrical portion 50A. Of these, the bulge portion 50B provides, at the front thereof, a face M1 at which various ports are set. At this setting face M1, a catheter-inserting port 52 and a therapeutic-instrument inserting port 53 are formed to protrude and open outside, and allow the foregoing catheter 12 and therapeutic instrument 13 to be inserted through the overtube 11. Concretely, the catheter 12 is to be Inserted Into the one, 52, of the Inserting ports 52 and 53, while various types of therapeutic instruments 13 are to be inserted Into the remaining inserting port 53.

The therapeutic-instrument inserting port 53 is formed as a columnar member protruding from the setting face M1. This columnar member, i.e., the Inserting port 53, has a given inner diameter and is formed to communicate with the inner channel 23 of the overtube 11. Thus an operator is able to insert the therapeutic instrument 13 Into this Inserting port 53, resulting in feeding the therapeutic instrument 13 through the inner channel 23 in free forward/backward movements.

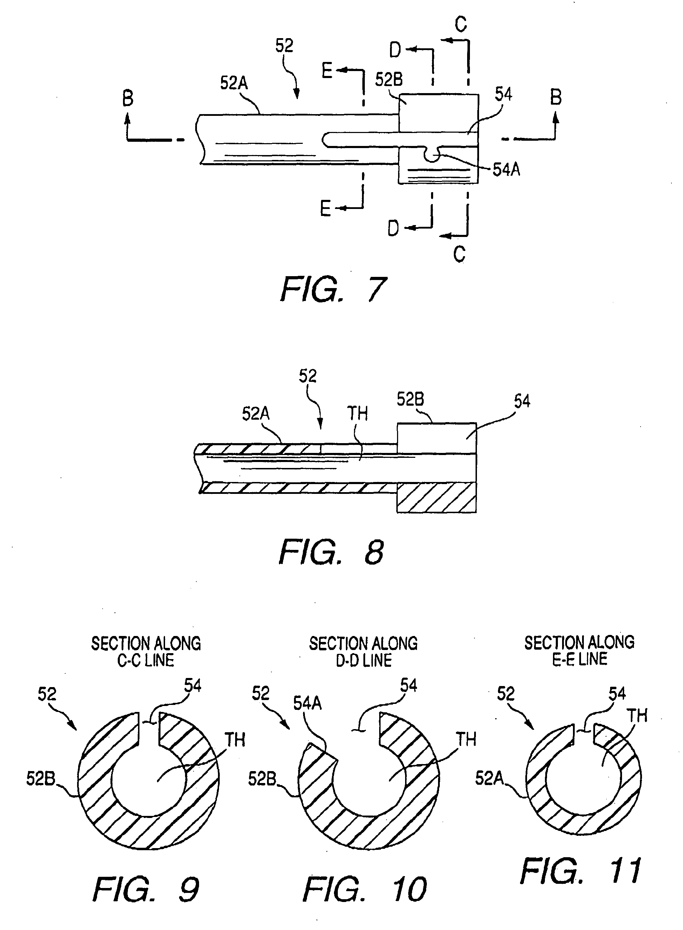

In a similar way to the above, the catheter-inserting port 52 is a protruded columnar member formed at the setting face M1. The axial length of this columnar member, i.e., the inserting port 52, is set to be longer than that of the therapeutic-instrument inserting port 53. As shown in Figs. 7-11, this inserting port 52 consists of a distal end portion 52A extended from the setting face M1 and a base end portion 52B crowned at the base-side end of the distal end portion 52A. The base end portion 52B is larger in outer diameter than the distal end portion 52A. Through both the distal end portion 52A and the base end portion 52B, there is formed a common inserting hole TH having an inner diameter which allows the catheter 12 to be inserted with a given clearance from the hole. In addition, as shown in the figures, through part of the distal end portion 52A and the base end portion 52B, a slit 54 with a predetermined length is formed along their axial direction. This slit 54 is formed to pass through the wall bodies of both the base end portion 52B and the distal end portion 52A. Moreover, at a halfway position of the slit 54, which is located within the base end portion 52B, a clearance groove 54A is formed to deviate laterally from the slit 54 and communicate with the slit 54. Hence an operator's insertion of the catheter 12 into this inserting port 52 makes it possible to feed the catheter 12 into the inner channel 24 in free forward/backward movements. With the catheter 12 inserted in the channel 24 via the inserting port 52, the clearance groove 54A can be used to lock the catheter 12 to the overtube 11 in a state where the catheter 12 and the overtube 11 are positioned, as to their distal ends, with each other in the axial direction. This locking operation will be detailed later again.

On and around the cylindrical portion 50A of the handheld operating portion 11D, there are provided a balloon air-supply/exhaust button 55, a suction button 57, a bendable-portion operating lever 58, a therapeutic-instrument elevating lever 59, and a catheter elevating lever 60. Of these components, the balloon air-supply/exhaust button 55 and the suction button 57 can be exemplified as electric switches to output an on/ff signal, respectively, and output lines of the switches are connected to the controller 15 via a universal cord 61. These buttons 55 and 57 may be formed as mechanical switches to mechanically switch ducts.

The remaining bendable-portion operating lever 58, therapeutic-instrument elevating lever 59, and catheter elevating lever 60 are formed as mechanically-operated operation devices.

Of these, the balloon air-supply/exhaust button 55 is a switch used to selectively command supply or exhaust of air into the balloon 20 or from the balloon 20 via the air-supply channel 41 (refer to Fig. 3C). This air-supply channel 41 is connected with the balloon controller 16 via a duct 63 in the handheld operating portion 11D, and this balloon controller 16 is in charge of supplying and exhausting air in response to a control signal from the controller 15. Incidentally, the medium used for expanding and shrinking the balloon 20 is not limited to gas, but may employ liquid.

The controller 15 is configured as, by way of example, a computer with a CPU and memories, in which the CPU operates on programs stored beforehand in the memories and is set to realize desired functions in response to operation signals from buttons and levers. Thus the controller 15 responds to a switch signal from the balloon air-supply/exhaust button 55 so as to supply to the balloon controller 16 control signals for commanding the supply and exhaust of the foregoing air.

In addition, the handheld operating portion 11D is provided with the suction button 57. Pressing this button 57 allows the balloon controller 16 to provide suction matters using the overtube 11.

When turning the bendable-portion operating lever, the rotation direction and rotation angle of the lever are mechanically transmitted to the bendable portion 11B via the wires. This allows the bendable portion 11B to be bent in the desired one of the up, down, right and left directions (when viewed axially) by a desired angle. In addition, this bendable-portion operating lever 58 Is provided with a lock mechanism (not shown), whereby the bendable portion 11B can be locked (fixed) at a desired bent angle.

Further, when the therapeutic-instrument elevating lever 59 is operated in the axial direction by rotation, its pulling operation force is mechanically transmitted to the elevating base 25 for therapeutic instruments via the wire 28. As a result, depending on the operated amount, the elevating base 25 is forced to be elevated toward the upright direction (in the radial direction) by an operator's desired angle. By contrast, when the elevating lever 59 is loosened in its rotation, the loosened operating force is mechanically transmitted to the elevating base 25 via the wires 28. Accordingly, depending on the operated amount, the elevating base 25 is subjected to a decrease in its elevation angle, returning the elevating base 25 in the axial direction. By operating the therapeutic-instrument elevating lever 59, an operator is able to freely adjust the elevation angle of the therapeutic instrument 13 in a given angular range.

Similarly, when the catheter elevating lever 60 is rotated in the radial direction, its pulling operation force is mechanically transmitted to the elevating base 26 for the catheter via the wire 31. Thus depending on the operated amount, the elevating base 26 is elevated upright (in the radial direction) by the desired angle. In contrast, when the elevating lever 60 is loosened from its rotated state, the loosened operation force is transmitted to the elevating base 26 via the wire 31. In response to the operated amount, the elevation angle of the elevating base 26 is reduced, returning the elevating base 26 to its axial direction. It is therefore possible for an operator to manipulate the catheter elevating lever 60 in order to arbitrarily adjust the elevation angle of the catheter 12 in a given angular range.

The upper limits of the elevation angles of the therapeutic instrument 13 and catheter 12 are decided by the axial positions of the rear ends of the slits 21A and 22A of both openings 21 and 22, respectively. In the present embodiment, as shown from Figs. 3A and 3B, the rear ends of the slits 21A and 22B are formed as inclined faces, respectively, so that the rear ends are located nearer to the base end side by a given distance L than the axial positions of the fixing pins 27 and 30, respectively. Hence the elevation angle ranges from 0 degrees (no elevation) to α degrees (larger than 90 degrees).

### (Catheter)

The catheter 12 will now be described.

The catheter 12 according to the present embodiment is used with insertion through the catheter channel 24 formed through the foregoing overtube 11 and is a flexible tubular member in which an imaging device is located for observing the inside of an object in forward viewing. Like the foregoing overtube 11, this catheter 12 is composed of a distal end portion 12A, a bendable portion 12B, a flexible tubular portion 12C, and a handheld operating portion 12D, which are lined up, as an integral member, from its distal side to its base side.

Of these, the distal end portion 12A is a rigid predetermined-length member located on the distal side of the catheter 12. Inside this distal end portion 12A, a CCD camera 71 serving as an imaging device and an LED 72 are embedded. Control lines and output lines for driving the CCD camera 71 are wired to axially penetrate through the bendable portion 12B and flexible tubular portion 12C, and handed from the handheld operating portion 12D to the controller 15 via a universal cord 73. Then the output signals from the CCD camera 71 are subjected to signal processing at a video processor (not shown) embedded in the controller 15, and then displayed by a monitor 74. This CCD camera 71 is usable for forward viewing and side viewing.

In addition, the controller 15 is equipped with a light source device (not shown). Power from the light device is transmitted to the handheld operating portion 12D through a universal cord 73, and transmitted to the LED 72 via a power line wired through the bendable portion 12B and flexible tubular portion 12C. Hence the LED 72 is able to emit light for illumination. This illuminating means is not necessarily an LED, but may be an optical fiber, for example.

The bendable portion 12B is located to integrally unite both the distal end portion 12A and the flexible tubular portion 12C. This bendable portion 12B responds to commands from the handheld operating portion 12D so that the bendable portion 12B is bent freely in any one of the four directions, i.e., the up, down, right and left directions (viewed axially). This active bendable operation requires pulling operations and loosening operations, and these operations are realized by, for example, not-shown operating wires connecting an operation lever 75 attached to the handheld operating portion 12D and the bendable portion 12B. Rotating the operation lever 75 causes the operating wires to be pushed and pulled, thus bending the bendable portion 11B in the desired direction.

The flexible tubular portion 12C is a flexible tubular member. The handheld operating portion 12D is an approximately columnar member having a size appropriate for grasping by the operator and serves as a base section of the flexible tubular portion 12C. This handheld operating portion 12D is equipped with an operation lever 75 and an air-supply water-supply button 77 which are for manipulating the bendable portion 12B. Therefore, when an operator turns the operation lever 75, the rotated direction and angle of the lever are transferred to the bendable portion 12B via wires, whereby the bendable portion 12B can be bent by the desired angle in the desired up, down, right or left direction (when axially viewed). This operation lever 75, has a lock mechanism (not shown), making it possible that the bendable portion 12B can be locked (fixed) at a desired bent angle. Moreover, an operator's use of the air-supply water-supply button 77 enables air and water to be supplied from the air-supply water-supply unit 17 by way of a cable 78. The air and water are fed to the distal end portion 12A via not-shown channels through the flexible tubular portion 12C and bendable portion 12B, and used to wash and dry an objective lens disposed in the distal end portion 12A and expand organs of the object being treated.

Further, at a base-side given position on the outer surface of the flexible tubular portion 12C, a protrusion 76 is formed. The size of the protrusion 76 is set such that the protrusion 76 is able to fit in the slit 54 and the clearance groove 54A formed through the foregoing inserting port 52. The catheter 12 is inserted completely through the inserting port 52 and then rotated therein, during which time the protrusion 76 is made to pass through the slit 54 and fit in the clearance groove 54A, thus being locked.

Incidentally the catheter 12 is not limited to the foregoing structure, but may be produced such that the catheter 12 has no active bendable portion, described above, and has only the distal end portion 12A and the flexible tubular portion 12C.

### (Approach to Vater's papilla for treatment of pancreatic duct and biliary duct)

The therapeutic system 1 according to the present embodiment is intended to perform a treatment on the pancreatic duct and biliary duct of a patient who had a Roux-en-Y reconstructive operation. Hence a doctor is to perform a therapeutic procedure such that the overtube 11 through which the catheter 12 is inserted is orally inserted into the small intestine of a patient.

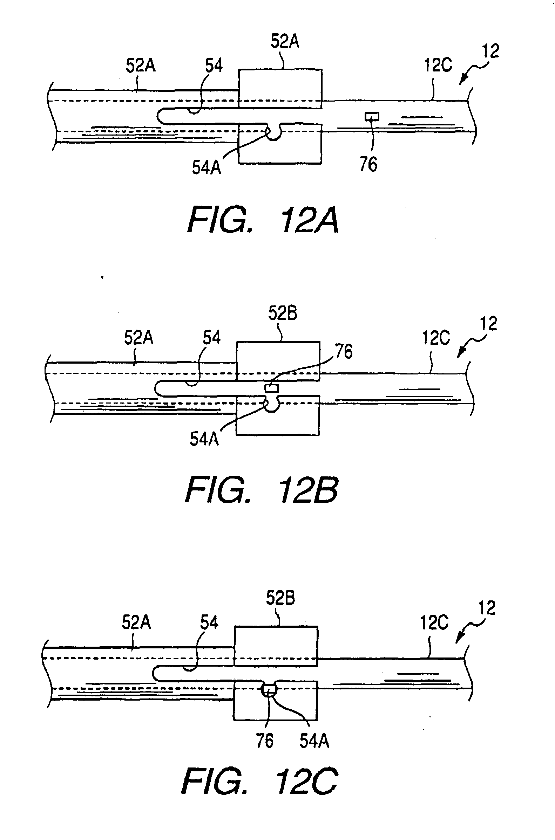

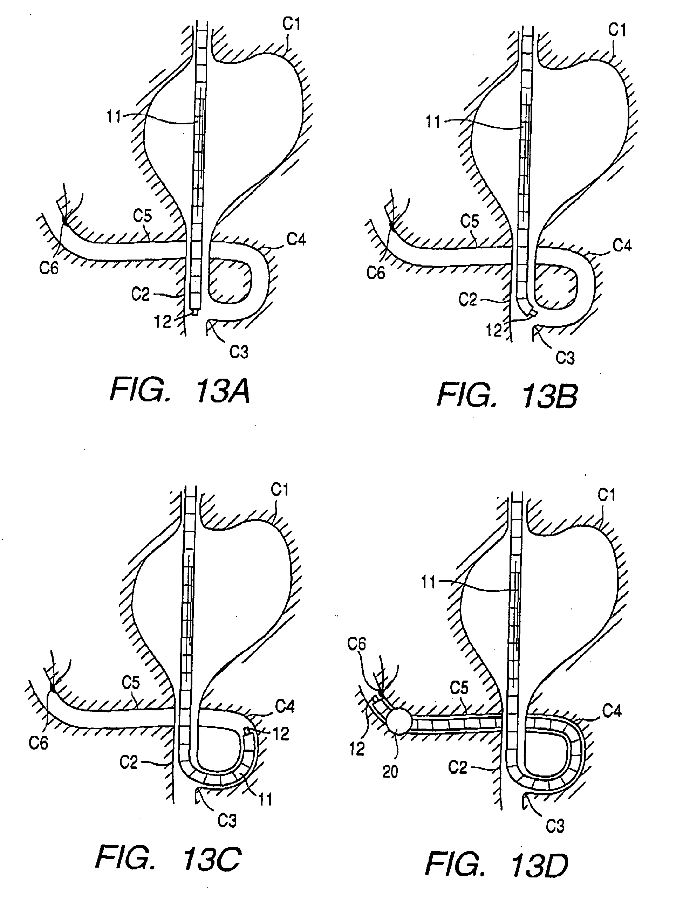

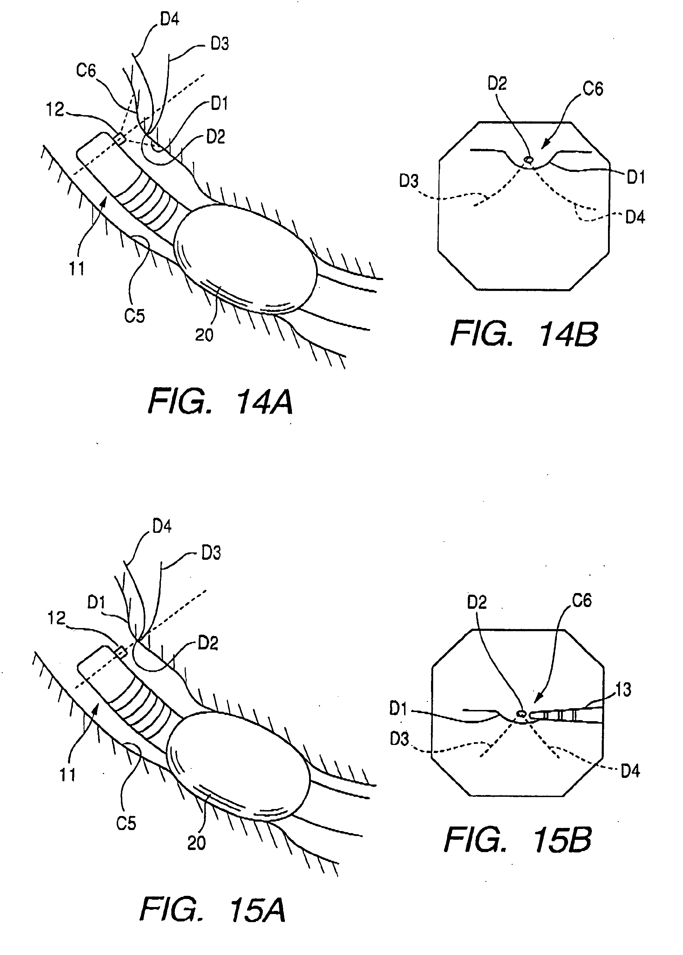

This therapeutic procedure will now be described.
(1) First, the catheter 12 is inserted into the catheter-inserting port 52 of the overtube 11 to pass through the catheter channel 24. During this insertion, the protrusion 76, formed at the base-side position on the catheter 12, is made to pass through the slit 54 of the inserting port 52. Then, when the protrusion 76 arrives at the position communicating with the clearance groove 54A of the slit 54, the catheter 12 is rotated to be directed toward the clearance groove 54A, whereby the protrusion 76 is fit into the clearance groove 54A (refer to Figs. 12A to 12C). This fitting action allows the catheter 12 to be locked to the overtube 11 and located about the initial position of the distal end of the catheter 12 and the Initial circumferential position of the catheter 12 (in the rotational directions).
   In the present embodiment, under the situation where the catheter 12 is at its initial position and locked to the overtube 11, the distal end of the catheter 12 reaches at least the distal end of the overtube 11, where the CCD camera 71 captures forward-viewing images of the forward view thereof. Those forward images are displayed by the monitor 74. The foregoing initial positioning makes it possible as well that the catheter 12 and the overtube 11 are located in their rotational directions such that their bendable portions 12B and 11B agree with each other in their up, down, right and left bending directions.
(2) Then the doctor orally inserts the overtube 11, through which the catheter 12 has been inserted, into the body of a patient, with observing images displayed on the monitor 74 (monitor images). In the patient body, this insertion allows the overtube 11 to pass the stomach C1 from the esophagus, and then enter the reconstructed jejunum.
(3) When the monitor images show that the distal end of the overtube 11 has arrived at a jejunum-to-jejunum anasto region C3 and is located at a given position facing the jejunum-to-jejunum anasto region C3 (refer to Fig. 13A), the doctor uses the operation lever 58 on the handheld operating portion 11D of the overtube 11 to bend the distal end portion 11A of the overtube 11 toward the jejunum-to-jejunum anasto region C3 (refer to Fig. 13B). Once this directional deflection is ended, it is not required to bend the bendable portion of the catheter 12 any more, so that it is desired to fix in place the bendable portion to avoid unintentional bent actions thereof.
(4) After this directional deflection, with observing the images on the monitor 74, the doctor continues to make the overtube 11 advance to arrive at a jejunum-anastomotic bent portion C4. Then, depending on the bent state of the jejunum-anastomotic bent portion C4, the doctor uses the operation lever 58 of the overtube 11 to adjust the direction of the distal end portion 11A of the overtube 11. With this directional adjustment done little by little, the overtube 11 is made to advance along the jejunum-anastomotic bent portion C4 (refer to Fig. 13C). On completion of passing along this jejunum-anastomotic bent portion C4, the doctor observes the monitor images to make the overtube 11 advance along the duodenum C5, and tries to find the Vater's papilla C6 (refer to Fig. 13D).
   By the way, when the overtube 11 is made to pass along the jejunum-to-jejunum anasto region C3 and the jejunum-anastomotic bent portion C4, the bendable portion 12B of the catheter 12 inserted in the overtube 11 may be bent together with the bendable portion 11B of the overtube 11 or may be bent subsidiarily, depending on the curvature of both the portions C3 and C4. This makes the bending actions smoother, making it possible that the jejunum-to-jejunum anasto region C3 and the jejunum-anastomotic bent portion C4 can be passed more smoothly and quickly.
(5) When the images displayed on the monitor 74 show a Vater's papilla C6 located in front of the catheter, the doctor uses the catheter elevating lever 60 to elevate the elevating base 26 for the catheter so that the distal end portion 12A of the catheter 12 stands approximately upright. Specifically, as illustrated in Fig. 14A, the distal end portion 12A is stood at approximately 90 degrees in respect to the axial direction of the distal end portion of the overtube 11. The monitor images in this situation are displayed as side views, as pictorially shown in Fig. 14B. Alternatively, the monitor images are not always confined to display a side view, but may be displayed as oblique views obtained at any angle in an angular range between the forward view (0 degree) and the side view (90 degrees).
   Provided that the Vater's papilla C6 is shown, the display images present therein a bulgy portion D1 and a papilla opening D2. In the figures, a reference D3 imaginarily shows a pancreatic duct and a reference D4 imaginarily shows a biliary duct.
   Hence, with observing the side-viewing images, during which time the doctor adjusts the overtube 11 as to its distal end position. For Instance, adjustment is made such that the papilla opening D2 is positioned at the approximate center of the monitor images.
(6) When this positing has been finished, the doctor uses the balloon air-supply/exhaust button 55 on the handheld operating portion 11D of the overtube 11 to expand the balloon 20. This expansion causes, as illustrated in Fig. 15A, the balloon 20 to forcibly push the wall of the duodenum C5, that is, to be attached firmly thereto, resulting in that a distal end section of the overtube 11, which includes the distal end portion 11A and bendable portion 11B, is fixed in place in the patient body by the balloon 20. In the case of Fig. 15A, the Vater's papilla D2 is present on the extended line from the axial line direction (axial direction) of the distal end portion 12A of the upright-standing catheter 12.
(7) The doctor then inserts the therapeutic instrument 13 Into the therapeutic-instrument inserting port 53 of the handheld operating portion 11D of the overtube 11. The instrument 13 is required for carrying out a treatment of the pancreatic duct and biliary duct. The overtube 11 has already reached the Vater's papilla C6 via the stomach C1, jejunum C2, jejunum-to-jejunum anasto region C3, jejunum-anastomotic bent portion C4, and duodenum C5. Hence a simple inserting operation of the therapeutic instrument 13 into the therapeutic-instrument channel 23 of the overtube 11 allows its distal end section to be guided to the Vater's papilla C6 smoothly.
(8) When the insertion of this therapeutic instrument 13 has been finished, the doctor then proceeds to use the therapeutic-instrument elevating lever 59 on the handheld operating portion 11D in order to elevate the remaining elevating base 25, bending (elevating) the distal end section of the therapeutic instrument 13 from its axial direction to the radial direction. In the present example, the angle of the therapeutic instrument 13, which is to be bend, i.e., the elevation angle of the elevating base 25, is manually set at approximately 90 degrees, in a similar way to the catheter 12 (that is, the catheter bending angle - the therapeutic-instrument bending angle = approx. 90 degrees). Fig. 15B exemplifies images displayed on the monitor 74 in such a situation.
   As Illustrated therein, the positioning has been done such that the Vater's papilla opening D2 is present in the axial direction of the distal end portion 12A of the catheter 12. Accordingly, provided that the elevation angle of the therapeutic instrument 13 is set to that of the catheter, the therapeutic instrument 13 is allowed to approach to the Vater's papilla opening D2.
(9) The doctor then makes the therapeutic instrument 13 reach the Vater's papilla opening D2, together with viewing the monitor 74, for performing the necessary pancreatic duct and biliary duct treatment.
(10) When the pancreatic duct and biliary duct treatment is completed, retracing the therapeutic instrument 13, returning the therapeutic Instrument 13 from its elevated state, pulling the therapeutic instrument 13, and returning the catheter 12 from its elevated state follow in sequence, before shrinking the balloon 20. The overtube 11 is then pulled out.

By the way, when the balloon 20 is expanded for being fixed in the body, there is a possibility that the Vater's papilla opening D2 is not on the extended line of the axial line direction (axial line) of the distal end portion 12A of the upright standing catheter 12, as exemplified in Fig. 16A. That is, the axial line direction F1 and the Vater's papilla opening D2 differ from each other in their positions, in which the axial line direction F1, i.e., the CCD camera 71 passes over the Vater's papilla D2 and is located far from the Vater's papilla. In this case, If the elevation angle of the therapeutic instrument 13 is the same as that of the catheter 12 (approx. 90 degrees), the approach to the Vater's papilla opening D2 cannot be done. To cope with this difficulty, the elevating base 25 is manually controlled in its elevation angle such that the therapeutic instrument 13 is bent more than 90 degrees. In other words, controls are set to achieve a situation where the bent angle (approx. 90 degrees) of the catheter < the bent angle of the therapeutic instrument. An image obtained in this manual control can be exemplified as shown in Fig. 16B, and the therapeutic instrument can approach the Vater's papilla opening D2.

### (Advantages)

In the present embodiment, as described, the catheter 12 with the CCD camera 71 (i.e., imaging device) mounted at the distal end thereof is Inserted through the overtube 11. During the insertion, the foregoing lock mechanism (composed of the protrusion 76, slit 54 and clearance groove 54A) enables the catheter 12 to be locked in a given attitude which holds a predetermined relative positional relationship with the overtube 11.

In this way, with the catheter 12 inserted through the overtube 11, the images acquired by the CCD camera 71 can be used as a guide to insert the overtube 11 into the patient's body. Since the catheter 12 is locked with the overtube 11, inserting the overtube 11 into the body causes the catheter 12 to be inserted concurrently into the body. Hence the doctor can be devoted to the insertion of the catheter, easing the doctor's insertion work.

In performing the insertion, under the forward viewing with the use of the CCD camera 71, the overtube 11 can be passed smoothly through the jejunum-to-jejunum anasto region and jejunum-anastomotic bent portion by bending operations of the overtube. When the targeted Vater's papilla C6 is reached, the balloon 20 is expanded to positionally fix the distal end section of the overtube 11, whereby the overtube, that is, the catheter 12 and the therapeutic instrument 13 are both stable as to their positions in the patient's body. In addition, the overtube 11 and catheter 12 can be prevented from coming away from the duodenum C5 during the treatment.

In this stably positioned state, the catheter 12 is bent (i.e., the elevating base 26 is elevated) to acquire side-viewing images from the CCD camera 71. Under this side viewing, the therapeutic instrument 13 is Inserted into the overtube 11. Since the overtube 11 has reached the Vater's papilla C6, the therapeutic instrument 13 can be made to advance to the Vater's papilla C6. Then the distal end section of the therapeutic instrument 13 can be bent (i.e., the elevating base 25 can be elevated). This bent angle can be adjusted by adjusting the elevation angle of the elevating base 25, depending on the positional relationship between the axial line direction of the distal end section of the catheter 12 and the Vater's papilla opening D2. Specifically, it is desired that the distal end section of the catheter 12 be bent to acquire side-viewing images by approximately 90 degrees relative to the axial line direction of the catheter body. In this way, even when the Vater's papilla opening D2 does not exist in the axial line direction of the distal end section of the catheter, the elevating base 25 can be adjusted in its elevation angle, whereby the bent angle can be set to a desired angle before or after approximately 90 degrees. Hence the balloon 20 is positionally fixed, so that in this fixed state, even if there is caused a positional shift between the CCD camera 71 and the Vater's papilla opening D2 in the axial direction of the catheter, the foregoing angle adjustment absorbs the positional shift to enable the therapeutic instrument 13 to approach the Vater's papilla opening D2.

In consequence, the present embodiment is able to provide an approach procedure which is effective for diagnosis of the pancreatic duct, the biliary duct, and the tissue of the Vater's papilla C6 and for the treatment of the pancreatic duct and biliary duct.

Additionally, as described above, combining the catheter 12 with the overtube 11, it is possible to change the observation view from the forward viewing during the insertion to the side viewing during the diagnosis and treatment, though the catheter 12 is only one in number. Thus the general versatility of the catheter can be enhanced.

### (Modifications)

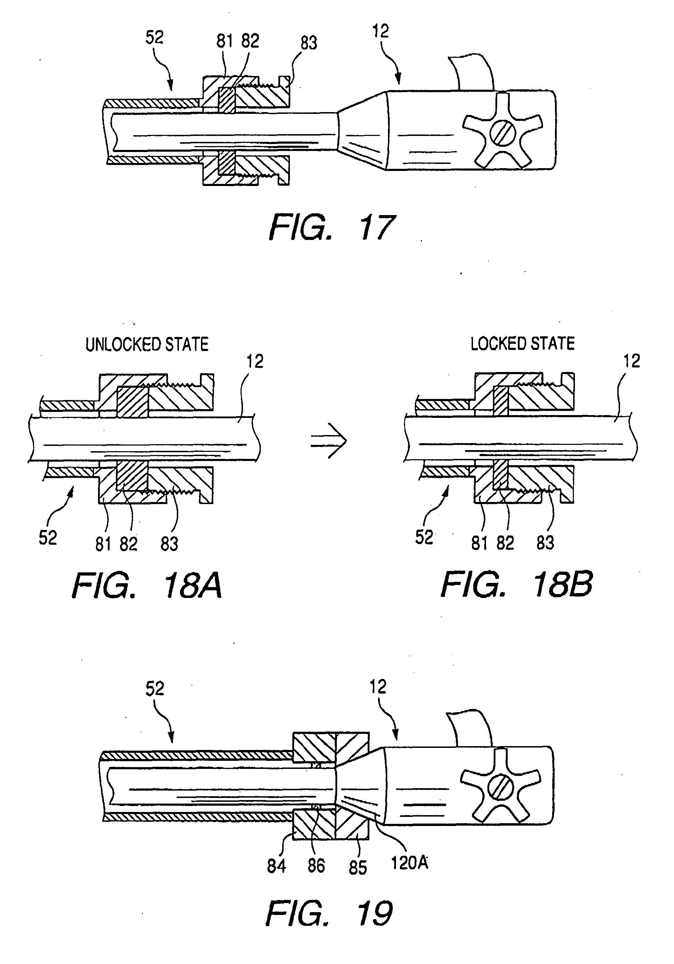

Referring to Figs. 17 - 19, modifications of the lock mechanism employed in the foregoing embodiment will now be described. Those modifications are formed to be replacements for the lock mechanism the protrusion 76, slit 54 and clearance groove 54A.

Figs. 17, 18A and 18B show a first modification of the lock mechanism. This modification relates to a mechanism locking the catheter 12 in the catheter-inserting port 52 by a screw. A cap 81 having a larger diameter than the port 52 is integrally attached to the distal end of this inserting port 52. An elastic ring 82 is placed inside the cap 81, where the elastic ring 82 is given a higher friction coefficient to the outer surface of the flexible tubular portion 13C of the catheter 13. This elastic ring 82 is formed to be pressed detachably by a knob 83 engaging with a thread on the inner circumferential surface of the cap 81.

Thus, as shown in Fig. 18A, with the knob 83 loosened, the catheter 12 is inserted into the catheter-inserting port 52 to put it through the catheter channel 24. In this inserted situation, the elastic ring 82 is not pressed, where the inner diameter of this elastic ring 82 is large, that is, kept at its predetermined value, so that the catheter 12 can be passed through the inserting port 52. Then the knob 83 is tightened, with the result that, as shown in Fig. 18B, the inner diameter of the elastic ring 82 is forced to shrink to allow its Inner-diameter portion to press part of the outer surface of the catheter 12. Hence the catheter 12 can be locked at a desired axial position relative to the overtube 11.

As a result, the catheter 12 can be locked at a desired position relative to the overtube 11.

Fig. 19 shows another modification of the lock mechanism. In this modification, at the end of the catheter-inserting port 52, a ring 84 and a grip 85 made of resin material such as elastomer are secured so as to be integrally connected in series. On the inner circumferential surface of the ring 84, a valve 86 for airtightness is secured. The grip 85 has an inner circumferential surface which is sloped to be widened gradually toward the outside. As part of the handheld operating portion 12D of the catheter 12, there is provided a breaking-preventing section 120A rigidly connecting both portions 12C and 12D and formed to have an outer surface which is larger in the slope angle than the inner surface of the ring 85. Setting is made such that, when the catheter 12 is fixed in the overtube, the distal end portion 12A of the catheter 12 is located outside the distal end portion 11A of the overtube 11, that is, protruded therefrom, or Is located at least at the same axial position as that of the overtube in either a forward-viewing state or a side-viewing state.

Hence, by forcibly inserting the catheter 12 into the inserting port 52 with the valve 86 touched during the insertion, the breaking-preventing section 120A can be pressed and fixed to the inner circumferential surface of the ring 85. Therefore it is possible to lock the catheter 12 to the overtube 11.

Moreover, as a third modification, there is provided a mechanism for expanding and shrinking the balloon 20. This mechanism is not always limited to the configuration that uses the foregoing balloon controller. For example, a syringe may be used.

Moreover, a fourth modification is provided, which is another example concerning with the procedure for approaching the Vater's papilla necessary during the foregoing treatment of pancreatic duct and biliary duct. Practically, this modification is concerned with, of the steps of this procedure, the steps shown in Figs. 13A and Fig. 13B described already. In the foregoing embodiment, a doctor handles the bendable-portion operating lever 58 of the overtube 11 to bend the distal end portion 12A of the catheter 12 toward the jejunum-to-jejunum anasto region C3. This way can be modified further as follows. When the monitor images represents a situation where the distal end of the overtube 11 captures the jejunum-to-jejunum anasto region C3 such that the distal end arrives at a given position facing the portion C3 (refer to Fig. 13A), the doctor uses the operation lever 75 of the handheld operating portion 12D of the catheter 12 so as to bend, in the direction, the distal end portion 12A of the catheter 12 toward the jejunum-to-jejunum anasto region C3 (refer to Fig. 13B). In association with this bending action, the distal end portion 11A of the overtube 11 is deflected together with that of the catheter toward the jejunum-to-jejunum anasto region C3. In this way, similarly to the foregoing embodiment, the directions can be changed easily.

### (Second embodiment)

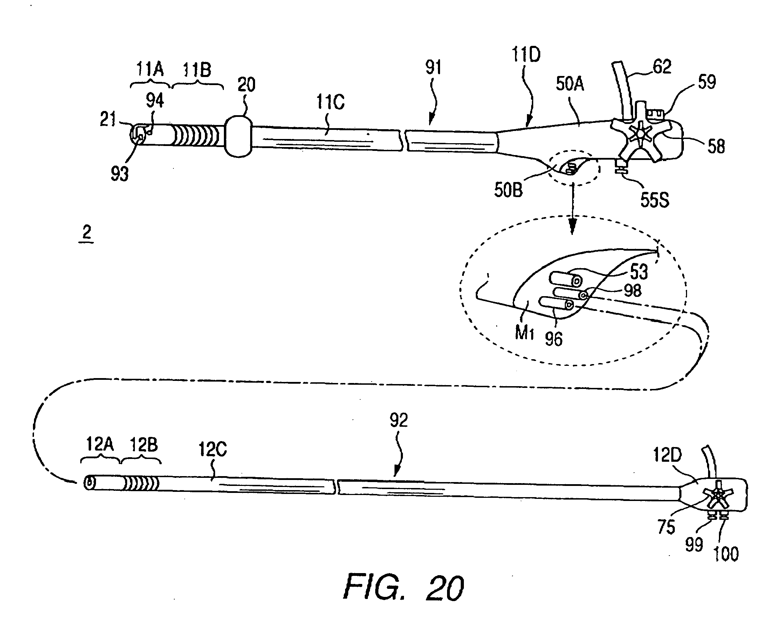

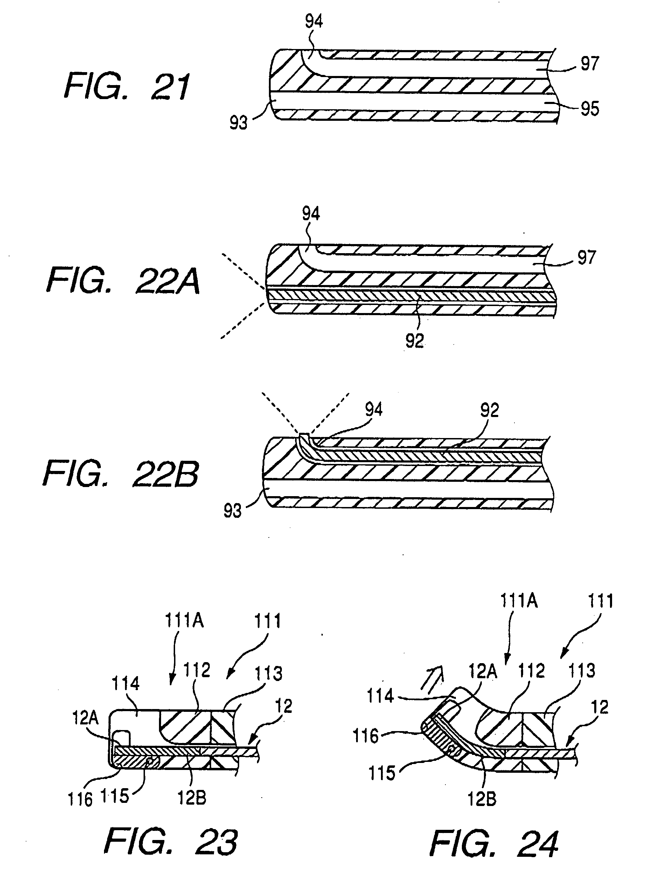

Referring to Figs. 20 - 22A and 22B, a second embodiment of the therapeutic system according to the present invention will now be described. In the following description, the same or identical components as or to that in the foregoing first embodiment are given the same reference numerals for the sake of an omitted or simplified explanation.

A therapeutic system 2 according to the second embodiment is provided with an overtube 91 structured similarly to the foregoing except for a catheter channel and a catheter 92 equipped with the CCD camera 71 serving as the imaging device. And the therapeutic system 2 is characteristic of making the CCD camera 71 of this same catheter 92 usable in both the forward-viewing and the side-viewing.

Like the first embodiment, the overtube 91 is provided with the distal end portion, bendable portion 11B, flexible tubular portion 11C, and handheld operating portion 11D. In the distal end portion 11A, in addition to the foregoing therapeutic-Instrument opening 21, as shown in Fig. 21, there are formed therealong a distal-end opening 93 and a side opening 94 both serving as openings for a catheter.

Of these, the distal-end opening 93 is formed to communicate with a first catheter channel 95, and this first catheter channel 95 continuously passes through the bendable portion 11B and the flexible tubular portion 11C so as to reach the setting face M1 of the bulge portion 50B of the handheld operating portion 11D. This setting face M1 has a forward-viewing inserting port 96 formed thereat, with which the first catheter channel 95 communicates. Thus the forward-viewing inserting port 96 and the distal-end opening 93 are formed to communicate with each other.

In addition, at a given position of the side of the distal end portion 11A, which position is close to the distal-end opening 93, there is formed a side opening 94 opened to the outside (at an angle of approx. 90 degrees with respect to the axial direction of the overtube). This side opening 94 is formed to communicate with a second catheter channel 97, which is, as understood from Fig. 22B, is guided to open radially and is bent in its inside to be along the axial direction. In this way, the second catheter channel 97 continuously passes the bendable portion 11B and the flexile tubular portion 11C and reaches the setting face M1 of the budge portion 50B of the handheld operating portion 11D. A side-viewing inserting port 98 is formed at the setting face M1 and the second catheter channel 97 is formed to communicate with this side-viewing inserting port 98. Hence the side-viewing inserting port 98 and the side opening 94 communicate with each other.

Furthermore, the handheld operating portion 11D is provided with, like the foregoing, the bendable-portion operating lever 58, therapeutic-instrument elevating lever 59, and balloon air-supply/exhaust button 55.

Meanwhile, like the foregoing first embodiment, the catheter 92 is provided with the distal end portion 12A, bendable portion 12B, flexible tubular portion 12C, and handheld operating portion 12D. In the distal end portion 12A, the CCD camera 71 is embedded so that its viewing is directed forward along the axial direction. In addition to the operation lever 75, the handheld operating portion 12D is provided with an air-supply water-supply button 99 and a suction button 100.

Accordingly, in the structures according to the second embodiment, the overtube 91 has no suction function, but instead of it, the catheter 92 has the suction function.

The overtube 91 and catheter 92 are structured in the similar or same way to or as those of the first embodiment.

The therapeutic system 2 thus constructed is orally inserted into a Roux-en-Y Gastrectomy patient to conduct the treatment of the pancreatic duct and biliary duct of the patient. When this insertion is made, the catheter 92 is inserted from the forward-viewing inserting port 96 into the first catheter channel 95, and its distal end is located at the distal-end opening 93 of the overtube 91, as shown in Fig. 22A, in the guide step in which the overtube 91 is made to advance to arrive at a position near to the Vater's papilla C6. During this guide step, the monitor screen represents images acquired under the forward viewing.

After the overtube 91 arrives in the position near the Vater's papilla C6, the doctor pulls out the catheter 92 from the overtube 91. Then the doctor re-inserts the catheter 92 into the second catheter channel 97 from the side-viewing inserting port 98, during which operation the distal end of the catheter 92 is located at the side opening 94 of the overtube 91 (refer to Fig. 22B). As a result, the monitor screen is switched to represent images acquired under the side viewing, so that, with observing the side-viewing images, the doctor performs the precise positioning which is similar to the foregoing one, positional fixing by expanding the balloon 20, inserting and elevating (bending) the therapeutic instrument 13, and making the therapeutic instrument 13 approach the Vater's papilla opening D2 for the treatment of the pancreatic duct and biliary duct (steps for positionally fixing and treatment).

Accordingly, the present embodiment provides similar operations and advantages to those In the first embodiment, and additionally allows the catheter 92 with the CCD camera to be inserted into the two catheter channels 95 and 97 individually. It is thus possible to use the one catheter 92 in acquiring both forward-viewing images and side-viewing images In a switchable manner. Compared to the first embodiment, it is sufficient to have only one elevating base for the therapeutic instrument, simplifying the structures for the elevation such as elevating bases and wires.

### (Modifications)

A variety of modifications applicable to the first and/or second embodiments will now be described.

### (First modification)

Referring to Figs. 23 and 24, a first modification will now be described. The first modification relates to another structure of the distal end portion of the overtube and is application to the configuration described in the first embodiment.

As shown in Fig. 23, an overtube 111 has a distal end portion 111A, to which a distal-side-positioned flexible portion 112 and a base-side-positioned rigid portion 113, which are formed to have the same diameter and rigidly coupled with each other, are rigidly connected in the axial direction. That is, the overtube 111 has no active bendable portion, and instead, has the flexible portion 112 which can be bent passively. The flexible portion 112 is made of the same material (for example, resin materials such as polyurethane, silicone, nylon, or latex) as that of the rigid portion 113, but manufactured such that the flexible portion 112 is softer and less rigid than the rigid portion 113.

The flexible portion 112 is formed to allow a catheter opening 114 to be opened therefrom. In addition, like the foregoing, a therapeutic-instrument opening is also opened therefrom, but not shown in the figure.

In the catheter opening 114, like the foregoing embodiment, there is disposed an elevating base 116 revolving on a fixing pin 115. Incidentally, a wire to transmitting pulling and relaxing actions for the elevation is connected to the elevating base 116, though not shown. On this elevating base 116, the distal end portion 12A of the catheter 12, which accommodates therein the CCD camera 71, is mounted. When this mounting is used, the bendable portion 12B of the catheter 12 should be located adjacently to the wall of the flexible portion 112, which wall faces the opening 114. To keep this location, this wall position, that is, the position of the flexible portion 112, is defined previously.

Hence, when the operation lever 75 of the catheter 12 is operated, the bendable portion 12B can be bent, and a force on this bent action causes the flexible portion 112 of the overtube 111 to be bent responsibly, as shown In Fig. 24. By operator's operations for bending the catheter 12, which involve observation of monitor images capturing an operator's desired direction, it is possible not only to deflect the distal end portion 12A of the overtube 11 but also allow the catheter 12 and the overtube 11 to be inserted concurrently. This will lead to simplifying the operations which should be done by operators.

### (Second modification)

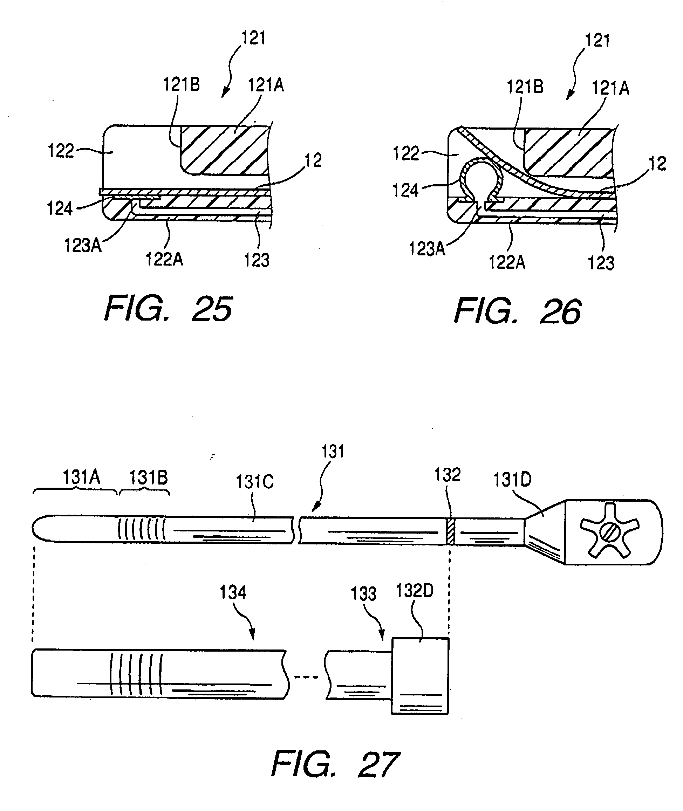

Referring to Figs. 25 and 26, a second modification will now be described. This second modification is concerned with another structure of the elevating base, which Is applicable to both the first and second embodiments.

As shown in Fig. 25, an overtube 121 has a distal end portion 121 with a catheter opening 122 formed therein. There is also formed a therapeutic-instrument opening in that portion, though not shown.

This opening 122 has a slit form made by cutting a corner of the distal end portion 121A to leave a bottom 122A and a wall 121B. In and along the bottom 122A, a fluid path 123 for transporting fluid and being connected to an external fluid source (not shown) is formed in the bottom 122A along the axial direction thereof. The base-side end of this path 123 is connected to an external conduit via a not-shown handheld operating portion and this conduit is coupled with the fluid source. In addition, the distal-side end of this path 123 is opened outward at the bottom 122A so as to form an opening 123A. On the bottom 122A, a balloon 124 is arranged to airtightly seal the opening 123A. Since the opening 123A is sealed by the balloon 124, feeding the fluid, such as normal saline, from the fluid source through the path 123 makes the balloon 124 expand in response to its fed pressure. In contrast, when the fluid is returned to the fluid source, the balloon 124 is made to shrink. The balloon 124 is made of resin materials, such as silicone resin or latex resin.

Accordingly, the balloon 124 can serve as the foregoing elevating base through its expansion and shrinkage. The distal end portion 12A of the catheter 12 is placed on the balloon 124 which is almost flat by its shrinkage, and in this attitude, the embedded CCD camera 71 provides forward-viewing images. When side-viewing images are needed, the balloon 124 is expanded as shown in Fig. 26. This expansion causes the distal end portion 12A of the catheter 12 to be elevated so as to provide side-viewing images. Depending on the location of the balloon 124 and an expanded size of the balloon 124, which is achieved by the supplied fluid, the elevation angle of the distal end portion 12A can be adjusted.

Hence, it is possible to provide similar operations and advantages to those in the foregoing embodiments and to additionally provide relatively lower manufacturing cost due to a reduced number of parts.

### (Third modification)

Referring to Fig. 27, a third modification will now be described. The present third modification concerns another structure of the elevating base as well. This modification is applicable to both the first and second embodiment.

Practically, the third embodiment concerns positioning both distal end portions of an overtube and a catheter. As shown in Fig. 27, a catheter 131 according to the present modification has a distal end portion 131A in which the CCD camera 71 is embedded as the imaging device. This catheter 131 has a flexible tubular portion 131C and a handheld operating portion 131D, and there is a marking 132 at a given position of the outer surface of the flexible tubular portion 131C, which given position is near the handheld operating portion 131D. This marking 132 is for example a color line applied on the surface or a groove formed on the surface. Incidentally, in Fig. 27, a reference 131B shows a bendable portion.

The position of the marking 132 is decided in such a manner that, when both distal end portions of the catheter 131 and an overtube 134 are positioned to agree with each other, the position corresponds to a length ranging from the distal end of the overtube 134 to an inlet of a catheter inserting port 133 formed in along a handheld operating portion 132D of the overtube. Thus, when the catheter 131 is inserted into the overtube 134 from its inserting port 133 until the marking 132 is made to positionally agree with the end face of the inserting port 133, i.e., the operating portion, the doctor recognizes that the distal end of the catheter 131 reaches the distal end of the overtube 134 and both distal ends positionally agrees with each other. That Is, It is understood that the given inserted state of the catheter is realized.

Hence it is possible to facilitate operations for positioning for the catheter 131 and the overtube 134 through which the catheter 131 is inserted.

### (Fourth modification)

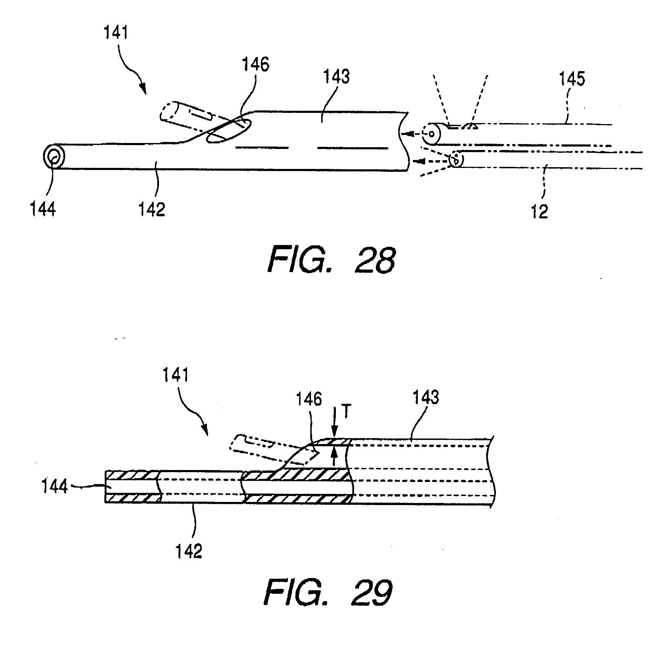

Referring to Figs. 28 and 29, a fourth modification will now be described. The fourth modification relates to a modified form of the overtube and is applicable to the first embodiment.

In Fig. 28, an overtube 141 according to the present modification is shown, in which the overtube 141 comprises a long distal end portion 141A which is formed into a shape resembling the nose of a mole. This distal end portion 141A includes a first extended part 142 which is a longer nozzle-like member and a second extended part 143 which is a shorter nozzle-like member and located at the upper part of the first extended part 142. Both the first and second extended parts 142 and 143 are produced as a single member.

The first extended portion 142 has a forward-viewing insertion channel 144 formed therethrough, in which the distal end portion 12A of the catheter 12, which distal end portion is equipped with the CCD camera 71, is inserted through the insertion channel 144. The distal end of the insertion channel 144 is opened outward in the axial direction thereof. Thus, using the catheter 12 Inserted through this insertion channel 144, the CCD camera 71 is able to provide forward-viewing Images.

Meanwhile the second extended part 143 has a side-viewing insertion channel 146 into which the insertion tube of a side-viewing endoscope 145 is inserted. The distal end of the insertion channel 146 is cut obliquely so as to be opened outward. When the insertion tube of the side-viewing endoscope 145 is inserted through this insertion channel 146, a not-shown bending lever of this endoscope 145 is operated, whereby the distal end of the insertion tube is made to protrude from the obliquely cut opening of the insertion channel 146 and can be bent obliquely along the opened direction of the oblique opening (refer to a dashed-two dotted line In Fig. 28). For making this bending operation easier, the insertion channel 146 has an opening-side positioned wall of which thickness T is thinner, as shown in Fig. 29.

Hence, only the catheter 12 is first inserted into the forward-viewing Insertion channel 144, and then the overtube 141 is made to approach to the Vater's papilla to be positionally fixed thereat. Then the side-viewing endoscope 145 is inserted into the side-viewing Insertion channel 146, before the distal end of the insertion tube is bent towards the papilla opening. The various functions of the side-viewing endoscope 145 are also used to perform the treatment of pancreatic duct and biliary duct. It is therefore possible to use the existing side-viewing endoscope 145 such that the forward-viewing using the catheter 12 and the side-viewing using the side-viewing endoscope 145 are switchable therebetween for the treatment of the pancreatic duct and biliary duct, as explained in the foregoing embodiment.

Incidentally, the scope of the present invention will not be limited to the configurations described in the foregoing embodiments and their various modifications, but the present invention may be reduced into practice in appropriate modes combined with conventional known structures, without departing from the scope of the present invention described in the appended claims.

## Claims

1. A therapeutic system comprising:
a catheter equipped with an imaging device outputting a signal based on imaging;
an overtube to be inserted into inside an object; and
a display displaying images based on the signal outputted from the imaging device,
wherein the overtube comprises a catheter channel through which the catheter Is allowed to be inserted and a deflecting device changing a direction of the catheter inserted through the catheter channel.

2. The therapeutic system of claim 1, wherein the deflecting device comprises
an elevating base formed In an opening located at a distal end of the catheter channel and formed to allow a distal end portion of the catheter to be mounted on the elevating base to elevate the distal end portion within an angular range made from an axial direction of the catheter channel to a radial direction perpendicular to the axial direction; and
an operation device to operate the elevating base.

3. The therapeutic system of claim 2, wherein the opening includes a slit communicating with the catheter channel and opening outside in an angular range ranging from the axial direction to, at least, the radial direction.

4. The therapeutic system of claim 1, wherein
the overtube includes a therapeutic-instrument channel thro ugh which the therapeutic instrument is inserted;
the deflecting device comprises
a further elevating base formed in an opening located at a distal end of the therapeutic-instrument channel and formed to allow a distal end portion of the therapeutic instrument to be mounted on the further elevating base to elevate the distal end portion within an angular range made from an axial direction of the therapeutic-instrument channel to a radial direction perpendicular to the axial direction; and
a further operation device to operate the further elevating base.

5. The therapeutic system of claim 4, wherein the opening includes a slit communicating with the therapeutic-instrument channel and opening outside in an angular range ranging from the axial direction to, at least, the radial direction.

6. The therapeutic system of claim 4, wherein
the opening at the distal end of the therapeutic-instrument channel is formed into a bottomed groove that has a slit-like form;
the elevating base is located at a bottom of the slit-like bottomed groove so that the elevating base is rotatable on a fixing axis; and
a size of the bottomed groove and a position of the fixing axis are set in the axial direction of the therapeutic-instrument channel so that the elevating base is given an elevation angle larger than 90 degrees.

7. The therapeutic system of claim 6, wherein
the opening at the distal end of the catheter channel is formed into a further bottomed groove that has a slit-like form;
the further elevating base is located at a bottom of the further bottomed groove so that the further elevating base is rotatable on a further fixing axis; and
a size of the further bottomed groove and a position of the further fixing axis are set in the axial direction of the catheter channel so that the further elevating base is given an elevation angle of at least 90 degrees.

8. The therapeutic system of claim 1, comprising a fixing function for positionally fixing the catheter to the catheter channel.

9. The therapeutic system of claim 1, wherein
the catheter comprises a bendable portion that is bendable In response to a controlling force and means for giving the controlling force to the bendable portion;
the catheter comprises a further bendable portion that is bendable; and
both bendable portions of the catheter and the overtube are present at approximately fixed positions in the axial direction of the catheter.

10. The therapeutic system of claim 1, wherein
the elevating base is a balloon driven depending on injection and ejection of fluid.

11. The therapeutic system of claim 1, wherein
the catheter channel includes a first duct, which is for forward viewing, extending along the axial direction of the overtube and being opened from a front face of the overtube and a second duct, which is for side viewing, extending along the axial direction of the overtube and being opened from a side surface perpendicular to the axial direction of the distal end at the distal end of the overtube.

12. The therapeutic system of claim 2, comprises
an actuator operating for positionally fixing the overtube within an object to be treated; and
control means for controlling the fixing operations of the actuator.

13. The therapeutic system of claim 12, wherein
the actuator is a balloon disposed on an outer circumferential surface of the overtube and expandable and shrinkable responsively to injection and ejection of fluid.

14. The therapeutic system of claim 13, wherein
the overtube comprises a balloon air-supply and air-discharge channel and a suction channel;
means for performing air supply and air discharge to and from the balloon through the balloon air-supply and air-discharge channel; and
means for performing suction through the suction channel.
